# EUROPÄISCHE PATENTSCHRIFT

(11) **EP 2 542 897 B1**
(45) Veröffentlichungstag und Bekanntmachung des Hinweises auf die Patenterteilung: **17.06.2015**
(21) Anmeldenummer: 11718256.8
(22) Anmeldetag: 02.03.2011
(51) Int. Cl.: G01N 33/68, H01J 49/04

(54) **Verwendung von halogenierten Cyanozimtsäurederivaten als Matrizes in MALDI-Massenspektrometrie**
Use of halogenated cyanocinnamic acid derivatives as matrices in MALDI mass spectrometry
Utilisation de dérivés halogénés d'acide cyanocinnamique en tant que matrice en spectrométrie de masse MALDI

(30) Priorität: 04.03.2010 DE 102010010155
(43) Veröffentlichungstag der Anmeldung: 09.01.2013
(73) Patentinhaber: Sigma-Aldrich International GmbH, 9000 St. Gallen (CH)
(72) Erfinder: JASKOLLA, Thorsten, 64331 Weiterstadt (DE); KARAS, Michael, 65795 Hattersheim (DE)
(74) Vertreter: Scholz, Volker
(86) Internationale Anmeldenummer: PCT/DE2011/000204
(87) Internationale Veröffentlichungsnummer: WO 2011/107076

(56) Entgegenhaltungen:
- WO-A1-2006/124003
- DE-B3-102007 040 251
- JP-A- 3 071 141
- US-A- 2 749 232
- JASKOLLA THORSTEN W ET AL: "4-Chloro-alpha-cyanocinnamic acid is an advanced, rationally designed MALDI matrix", PROCEEDINGS OF THE NATIONAL ACADEMY OF SCIENCES, NATIONAL ACADEMY OF SCIENCES, WASHINGTON, DC; US, Bd. 105, Nr. 34, 1. August 2008 (2008-08-01), Seiten 12200-12205, XP002504012, ISSN: 0027-8424, DOI: DOI:10.1073/PNAS.0803056105 in der Anmeldung erwähnt
- JASKOLLA T ET AL: "The new matrix 4-chloro-[alpha]-cyanocinnamic acid allows the detection of phosphatidylethanolamine chloramines by MALDI-TOF mass spectrometry", JOURNAL OF THE AMERICAN SOCIETY FOR MASS SPECTROMETRY ELSEVIER SCIENCE INC. USA, Bd. 20, Nr. 5, Mai 2009 (2009-05), Seiten 867-874, XP002647434, ISSN: 1044-0305 in der Anmeldung erwähnt
- TEUBER KRISTIN ET AL: "Significant sensitivity improvements by matrix optimization: a MALDI-TOF mass spectrometric study of lipids from hen egg yolk.", Juni 2010 (2010-06), CHEMISTRY AND PHYSICS OF LIPIDS JUN 2010 LNKD- PUBMED:20420816, VOL. 163, NR. 6, PAGE(S) 552 - 560, XP002647435, ISSN: 1873-2941 Zusammenfassung Abbildungen 1,2,3
- MARK F. WYATT ET AL: "Characterisation of organometallic and coordination compounds by solvent-free matrix-assisted laser desorption/ionisation time-of-flight mass spectrometry", THE ANALYST, vol. 133, no. 1, 1 January 2008 (2008-01-01), page 47, XP055105015, ISSN: 0003-2654, DOI: 10.1039/b713073b
- A. MARIE ET AL: "Fluorinated Matrix Approach for the Characterization of Hydrophobic Perfluoropolyethers by Matrix-Assisted Laser Desorption/Ionization Time-of-Flight MS", ANALYTICAL CHEMISTRY, vol. 75, no. 6, 1 March 2003 (2003-03-01), pages 1294-1299, XP055105016, ISSN: 0003-2700, DOI: 10.1021/ac0260802

## Beschreibung

Die vorliegende Erfindung betrifft die Verwendung eines halogenierten Cyanozimtsäurederivats als Matrix in der MALDI-Massenspektrometrie von Analyten sowie eine solche Matrix.

MALDI (Matrix-Assisted Laser Desorption/Ionization, Matrix-unterstützte Laser-Desorption/Ionisation) ist eine in den frühen 1980er Jahren entwickelte Technologie zur Ionisation verschiedener Molekülklassen. Dieses Verfahren beruht auf einer Inkorporation der zu untersuchenden Analyte in eine organische Matrix durch Kokristallisation. Nachfolgende Bestrahlung der kokristallisierten Probe mit einem Laser bewirkt Energieeintrag durch Absorption der Matrix und dient der zur Analyse notwendigen Desorption von einzelnen Molekülen und Molekül-Agglomeraten in die Gasphase sowie deren Fragmentierung zu den entsprechenden monomolekularen Spezies. Darüber hinaus bewirkt die Laserenergie (Photo)Ionisation der Matrix und der Analyte und ermöglicht dadurch deren separierte Detektion durch Beschleunigung der gebildeten Ionen in elektromagnetischen Feldern und Messung der massen- und Iadungsabhängigen Flugzeit. Durch die Inkorporation der Analyte in einem großen molaren Überschuss an Matrix wird ihre übermäßige Fragmentierung verhindert, so dass sich das MALDI-Verfahren nicht nur zur Detektion von kleineren Molekülen wie Arzneistoffen, Metaboliten oder Peptiden, sondern auch besonders gut zur Detektion von intakten großen und thermisch labilen Biomolekülen wie Proteinen, Oligonukleotiden oder auch z.B. von synthetischen Polymeren oder hochmolekularen anorganischen Verbindungen eignet.

Als Matrizes werden überwiegend kleine organische Moleküle verwendet, die bei der verwendeten Laserwellenlänge (überwiegend UV- oder IR-Laser) ausreichend stark absorbieren. Zusätzlich müssen verschiedene weitere Matrixanforderungen erfüllt sein, z. B. effizienter Analyteinbau in die Matrix durch Kokristallisation mit dieser, Analytseparierung innerhalb des Matrixkristalls, Vakuumstabilität, Löslichkeit in einem analyt-kompatiblen Lösungsmittel und hohe Analyt-Sensitivitäten. Übliche Matrizes weisen labile Protonen wie bei Carbonsäuren oder aciden Hydroxygruppen auf. Die bekannteste Matrixverbindung ist die überwiegend eingesetzte α-Cyano-4-hydroxyzimtsäure (CHCA oder HCCA). Weitere in der MALDI-MS Verwendung findende Matrizes sind zum Beispiel 4-Chloro-α-cyanozimtsäure (ClCCA), Sinapinsäure (4-Hydroxy-3,5-dimethoxyzimtsäure) oder 2,5-Dihydroxybenzoesäure (2,5-DHB). Unabhängig von ihrer spezifischen Struktur können Matrizes praktisch für alle mit MALDI-MS analysierbaren Substanzen eingesetzt werden, die unter anderem große und kleine sowie nicht flüchtige und thermisch labile Verbindungen umfassen, darunter Biomakromoleküle wie Proteine und Lipide, sowie organische und anorganische Analyte wie Arzneistoffe, Pflanzenmetabolite und dergleichen. Der gegenwärtige analytische Schwerpunkt liegt bei der Analyse von Peptiden.

Aus Beavis, R.C.; Chait, B.T. "Cinnamic Acid Derivatives as Matrices for Ultraviolet Laser Desorption Mass Spectrometry of Proteins", Rapid Communication in Mass Spectrometry (1989), Vol. 3, Nr. 12, Seiten 432-435 ist die Verwendung von hydroxy- bzw. methoxysubstituierten Zimtsäuren als Matrizes in der MALDI-Massenspektrometrie bekannt. Aus US 2002/0142982 A1 geht die entsprechende Verwendung von α-Cyanozimtsäure hervor. DE 101 58 860 A1 und DE 103 22 701 A1 beschreiben die Verwendung von α-Cyano-4-hydroxyzimtsäure bzw. 3,5-Dimethoxy-4-hydroxyzimtsäure. In DE 10 2007 040 251 A1 wird die Verwendung von halogenierten α-Cyanozimtsäuren beschrieben, aus Jaskolla et al. "4-Chloro-α-cyanocinnamic acid is an advanced, rationally designed MALDI matrix", Proc. Natl. Acad. Sci. USA (2008), Vol. 105, Nr. 34, Seiten 12200-12205, ist die Verwendung von ClCCA als MALDI-Matrix bekannt. Desweiteren offenbaren US 2006/0040334 A1 und WO 2006/124003 A1 MALDI-Matrizes auf der Basis von analyt-gekoppelten oder polymergekoppelten Derivaten der α-Cyano-4-hydroxyzimtsäure.

Durch sensitive Detektion deprotonierter Analyte im Negativ-Ionen Modus können verschiedene Vorteile für massenspektrometrische Analysen erzielt werden. So können über komplementäre Messungen sowohl des Positiv- als auch des Negativ-Ionen Modus zusätzliche Informationen über die Analyt-Beschaffenheit, wie bspw. die Analyt-Acidität, zugänglich werden, durch zusätzliche, nicht im Positiv-Ionen Modus detektierbare Signale können erhöhte Sequenzabdeckungen von z. B. Peptiden und Proteinen erzielt oder durch Analyse negativ geladener Analyt-Fragmente zusätzliche Strukturinformationen bei Fragmentierungen gewonnen werden. Darüber hinaus können verschiedene Analytklassen aufgrund fehlender basischer Gruppen und/oder Vorliegen leicht deprotonierbarer Funktionen nur schwer protoniert werden, wie beispielsweise phosphotyrosin-haltige Analyte (Bonewald et al. "Study on the synthesis and characterization of peptides containing phosphorylated tyrosine", Journal of Peptide Research (1999), Vol. 53, Nr. 2, Seiten 161-169), sulfatierte Analyte (Nabetani et al. "Analysis of acidic peptides with a matrix-assisted laser desorption/ionization mass spectrometry using positive and negative ion modes with additive monoammonium phosphate", Proteomics (2006), Vol. 6, Seiten 4456-4465), chlorierte Lipide mit wenig basischen Dichloramin-Kopfgruppen (Jaskolla et al. "The new matrix ClCCA allows the detection of phosphatidylethanolamine chloramines by MALDI-TOF MS", Journal of the American Society for Mass Spectrometry (2009), Band 20, Seiten 867-874) oder stark saure Peptide (Jainhuknan, J. und Cassady, C. J. "Negative ion postsource decay time-of-flight mass spectrometry of peptides containing acidic amino acid residues", Analytical Chemistry (1998), Vol. 70, Nr. 24, Seiten 5122-5128). Dies geht einher mit verminderten Sensitivitäten im Positiv-Ionen Modus und unterstreicht in Verbindung mit vorgenannten Möglichkeiten zusätzlicher Informationsgewinne die Notwendigkeit sensitiver Matrizes für den Negativ-Ionen Modus.

Die bislang bekannten Matrixverbindungen sind fast ausschließlich nur für die Detektion positiv geladener Ionen geeignet. Verbesserungen sowie rationale Ansätze zielen im ganz überwiegenden Maße auf Optimierungen der Sensitivität in dieser Polarität ab. Folglich wird für analytische Fragestellungen annäherend quantitativ der Positiv-Ionen Modus eingesetzt. Messungen im Negativ-Ionen Modus weisen im Vergleich zu den entsprechenden Positiv-Ionen-Messungen oftmals Abnahmen der Analyt-Signalintensitäten um mehrere Größenordnungen auf und sind daher vergleichsweise sporadisch in der wissenschaftlichen Literatur beschrieben. Die wenigen bekannten Ansätze einer Sensitivitätssteigerung im Negativ-Ionen Modus beschränken sich überwiegend auf Variation der Präparationsbedingungen und/oder Geräteparameter, wie z. B. Messungen im Linearmodus, in dem höhere Sensitivitäten durch ein drastisch verringertes Auflösungsvermögen erreicht werden (Janek et al. "Phosphopeptide analysis by positive and negative ion matrix-assisted laser desorption/ionization mass spectrometry", Rapid Communication in Mass Spectrometry (2001), Vol. 15, Seiten 1593-1599).

Die erzielbaren Sensitivitäten der MALDI-Massenspektrometrie für die Detektion und Nachweisempfindlichkeit positiver und negativer geladener Ionen sind gegenwärtig unbefriedigend und bedürfen weiterer Verbesserungen.

Aus der JP 3-071141 A ist die Verwendung von Cyanozimtsäurederivaten in einer elektrophotographisch sensitiven Beladungsschicht bekannt.

US 2,749,232 A offenbart Cyanozimtsäurederivate als Modulatoren des Pflanzenwachstums.

Es ist daher Aufgabe der vorliegenden Erfindung, durch Optimierung der Matrix-Struktur Verbindungen bereitzustellen, die für verschiedene Analyte, darunter die überwiegend unter-suchte Klasse der Peptide, durch signifikant erhöhte Analyt-Signalsensitivitäten im Positiv- und Negativ-Ionen Modus die Nachteile aus dem Stand der Technik zumindest teilweise überwinden.

Diese Aufgabe wird gelöst durch die Verwendung eines halogenierten Cyanozimtsäurederivats der allgemeinen Formel: 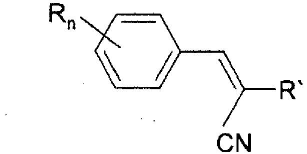 wobei R unabhängig ausgewählt ist aus F, Cl und Br, n = 3, 4 oder 5 ist und R' ausgewählt ist aus COOH, CONH₂, SO₃H und COOR" mit R" = C₁-C₅-Alkyl in einer Matrix für eine MALDI-Massenspektrometrie eines Analyten.

Erfindungsgemäß können sowohl cis- als auch trans-Isomere der halogenierten Cyanozimtsäurederivate eingesetzt werden. Ebenfalls können auch Konstitutions-Isomere der halogenierten Cyanozimtsäurederivate verwendet werden.

Sofern am Phenylring lediglich drei oder vier Halogensubstituenten vorhanden sind, sind die restlichen Phenylsubstituenten Wasserstoff. Die oben gezeigte Strukturformel soll alle denkbaren Konfigurationsisomere (cis/trans-Isomere) und Konstitutionsisomere (Stellungsisomere) der verschiedenen Cyanozimtsäurederivate einschließen. So soll beispielsweise ein Cyanozimtsäurederivat, das an den Positionen 2, 3 und 4 des Phenylrings substituiert ist, einem Cyanozimtsäurederivat entsprechen, das mit den gleichen Substituenten an den Positionen 4, 5 und 6 substituiert ist. Die Auswahl von F, Cl und Br als mögliche Substituenten ist völlig unabhängig voneinander, jedes R kann von einem anderen verschieden ausgewählt werden.

In einer besonders bevorzugten Ausführungsform wird R ausgewählt aus F, Cl und Br mit n = 5 und R' = COOH. Beispielhafte bevorzugte Verbindungen sind α-Cyano-2,3,4,5,6-pentafluorzimtsäure, α-Cyano-2,3,4,5,6-peniachlorzimtsäure und α-Cyano-2,3,4,5,6-pentabromzimtsäure.

In einer weiteren bevorzugten Ausführungsform wird R ausgewählt aus F, Cl und Br mit n = 4 und R' = COOH. Hier bevorzugte Verbindungen umfassen α-Cyano-2,3,4,5-tetrafluorzimtsäure, α-Cyano-2,3,4,5-tetrachlorzimtsäure und α-Cyano-2,3,4,5-tetrabromzimtsäure.

Weiterhin kann das Cyanozimtsäurederivat bevorzugt ausgewählt werden aus α-Cyano-2,3,5,6-tetrafluorzimtsäure, α-Cyano-2,3,5,6-tetrachlorzimtsäure und α-Cyano-2,3,5,6-tetrabromzimtsäure.

Bevorzugt sind ferner auch Cyanozimtsäurederivate, die ausgewählt sind aus α-Cyano-2,4,5,6-tetrafluorzimtsäure, α-Cyano-2,4,5,6-tetrachlorzimtsäure und α-Cyano-2,4,5,6-tetrabromzimtsäure.

Ebenfalls wird bevorzugt vorgeschlagen, dass R ausgewählt wird aus F, Cl, Br mit n = 3 und R' = COOH. Bevorzugte beispielhafte Verbindungen sind α-Cyano-2,3,4-trifluorzimtsäure, α-Cyano-2,3,4-trichlorzimtsäure, α-Cyano-2,3,4-tribromzimtsäure, α-Cyano-2,3,5-trifluorzimtsäure, α-Cyano-2,3,5-trichlorzimtsäure, α-Cyano-2,3,5-tubromzimtsäure, α-Cyano-2,3,6-trifluorzimtsäure, α-Cyano-2,3,6-trichlonimtsäure, α-Cyano-2,3,6-tribromzimtsäure, α-Cyano-2,4,5-trifluorzimtsäure, α-Cyano-2,4,5-trichlorzimlsäure, α-Cyano-2.4,5-tribromzimtsäure, α-Cyano-2,4,6-trifluorzimtsäure, α-Cyano-2,4,6-trichlorzimtsäure, α-Cyano-2,4,6-tribromzimtsäure, α-Cyano-3,4,5-trifluorzimtsäure, α-Cyano-3,4,5-trichlorzimtsäure und α-Cyano-3,4,5-tribromzimtsäure.

Ebenfalls besonders bevorzugt enthält die Matrix zusätzlich 4-Brom-α-cyanozimtsäure und/oder α-Cyano-2,4-dichlorzimtsäure.

Dabei ist bevorzugt, dass die Matrix zur MALDI-Massenspektrometrie negativer Ionen verwendet wird.

Besonders bevorzugt ist n = 5.

In einer Ausführungsform kann der Analyt ausgewählt sein aus Protein, Peptid, Polynukleinsäure, Lipid, phosphorylierter Verbindung, Saccharid, Arzneistoff, Metabolit, synthetischem und natürlichem (Ko)-Polymer und anorganischer Verbindung. Beispielhafte Analyte sind unter anderem Phosphopeptide oder Phospholipide, Dendrimere und Polynukleinsäuren, zum Beispiel DNA, RNA, siRNA, miRNA.

Ebenfalls wird bevorzugt vorgeschlagen, dass die Matrix mit dem Analyten vermischt wird.

Dabei kann das molare Mischungsverhältnis von Analyt zu Matrix 1:100 bis 1:1000000000, bevorzugt 1:10000, betragen.

Es können auch Mischungen von halogenierten Cyanozimtsäurederivaten, die unter die oben angegebene Formel fallen, verwendet werden.

Erfindungsgemäß am bevorzugtesten wird eine Mischung zumindest eines oben genannten halogenierten Cyanozimtsäurederivats mit zumindest einem weiteren Matrixmaterial eingesetzt.

Das weitere Matrixmaterial kann bevorzugt ausgewählt sein aus α-Cyano-4-hydroxyzimtsäure, α-Cyano-2,4-difluorzimtsäure, 2,5-Dihydroxybenzoesäure, Sinapinsäure, Ferulasäure, 2-Aza-5-thiothymin, 3-Hydroxypicolinsäure und 4-Chlor-α-cyanozimtsäure.

Werden erfindungsgemäß Mischungen mit weiteren Matrixmaterialien verwendet, werden die weiteren Matrixmaterialien in einem Anteil von 10 bis 90 Gewichtsprozent, vorzugsweise 20 bis 50 Gewichtsprozent, bezogen auf das Gesamtgewicht der Matrixmaterialien, eingesetzt. In einer weiteren bevorzugten Ausführungsform wird das Matrixmaterial mit einem inerten Füllstoff vermischt.

Auch kann die Matrix als ionische Flüssigkeit vorliegen.

Erfindungsgemäß ist ferner eine Matrix für eine MALDI-Massenspektrometrie eines Analyten, welche ein halogeniertes Cyanozimtsäurederivat der allgemeinen Formel: 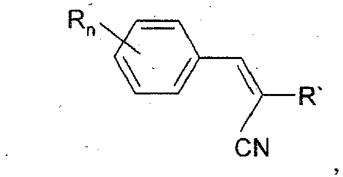 wobei R unabhängig ausgewählt ist aus F, Cl und Br, n = 3, 4 oder 5 ist und R' ausgewählt ist aus COOH, CONH₂, SO₃H und COOR" mit R'' = C₁-C₅=Alkyl; umfasst.

In einer bevorzugten Ausführungsform liegt die Matrix als ionische Flüssigkeit vor. Zur Herstellung einer ionischen Flüssigkeit kann die Matrix mit protonierbaren Basen, wie beispielsweise Pyridin, Diethylamin oder 3-Aminochinolin, versetzt werden, wodurch eine ionische Matrixlösung gebildet wird, die mit Analytlösungen vermischt oder als Film auf zu untersuchenden Oberflächen appliziert werden kann.

In einer weiteren Ausführungsform kann die Matrix in einem Lösungsmittel mit niedrigem Dampfdruck, zum Beispiel Glycerin, gelöst und nachfolgend mit dem gelösten Analyten vermischt oder als Film auf zu untersuchende Oberflächen appliziert werden.

Zur Durchführung der MALDI-Massenspektrometrie wird bevorzugt ein IR-Laser, UV-Laser, wie Nd:YAG- oder Stickstoff-Laser sowie Laser emitierend im mittleren oder fernen UV, wie durchstimmbare Farbstoff- oder optisch parametrische Oszillator (OPO)-Laser und 308 nm XeCl-Eximerlaser zum Energieeintrag verwendet.

Überraschenderweise wurde erfindungsgemäß festgestellt, dass bei Einsatz mehrfach halogenierter α-Cyanozimtsäurederivate in einer Matrix in der MALDI-Massenspektrometrie die Detektion und Nachweisempfindlichkeit für positive und negative Ionen, bevorzugt negative Ionen, deutlich verbessert werden kann. Der Einsatz der Derivate führt zu deutlich stärkeren Analytsignalintensitäten und Sensitivitäten im Negativ-Ionen-Modus als bisheriger Matrizes.

Ein Einsatz von Matrix-Mischungen, gemäß einer bevorzugten Ausführungsform, erlaubt eine Optimierung der Eigenschaften im Hinblick auf die Kristallisation und den Einbau von Analytverbindungen in die Matrixkristalle sowie eine effizientere Ionisation der Analyte.

Ohne an irgendeine Theorie gebunden werden zu möchten, wird angenommen, dass die Vorteile aufgrund der Auswahl elektronenziehender sowie leicht polarisierbarer Substituenten vorliegen, unabhängig von der exakten Positionierung und Anzahl der Halogensubstituenten am Aromaten.

Es wird angenommen, dass durch Einsatz der erfindungsgemäß vorgeschlagenen halogenierten α-Cyano-Zimtsäurederivate die folgenden Vorteile realisiert werden: Durch den ausgeprägten -I-Effekt stark elektronenziehender Halogene wird die hohe negative Ladungsdichte von im Verlauf sekundärer Ionisationsprozesse gebildeter Matrix-Carbanionen vermindert und damit deren Stabilität erhöht; in den neutralen Matrizes bewirken elektronenziehende Substituenten eine Verminderung der negativen Ladungsdichte der jeweiligen Matrix-π-Systeme, wodurch die bei der Photoionisation der Matrix oder des metallischen Substrats frei werdenden und ebenfalls negativ geladenen Elektronen zur Absorption durch weitere neutrale Matrix-Moleküle eine geringere Abstoßung überwinden müssen, resultierend in einer höheren Elektronenaffinität beziehungsweise Einfangwahrscheinlichkeit; steigt die Elektronenaffinität durch die Derivatisierung mit Halogensubstituenten an, so liegt mehr innere Energie zur Ausbildung reaktiver Carbanionen vor.

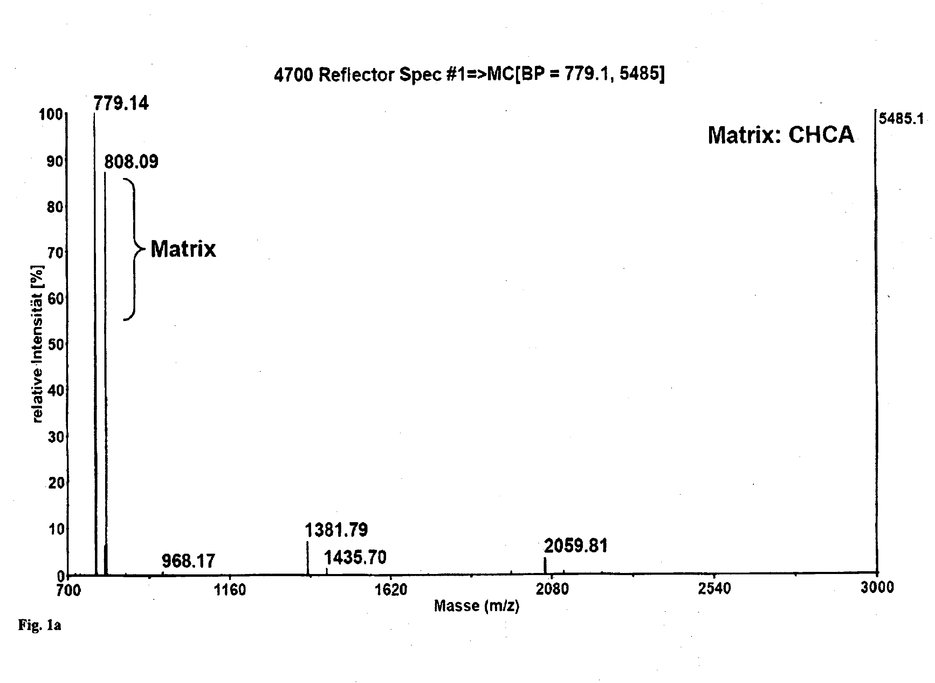

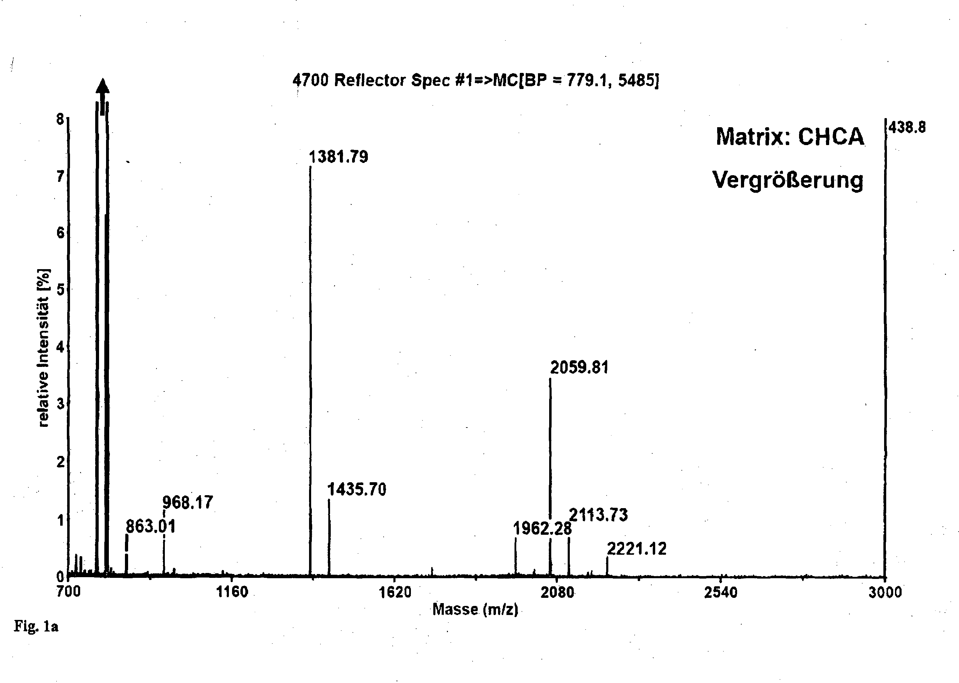

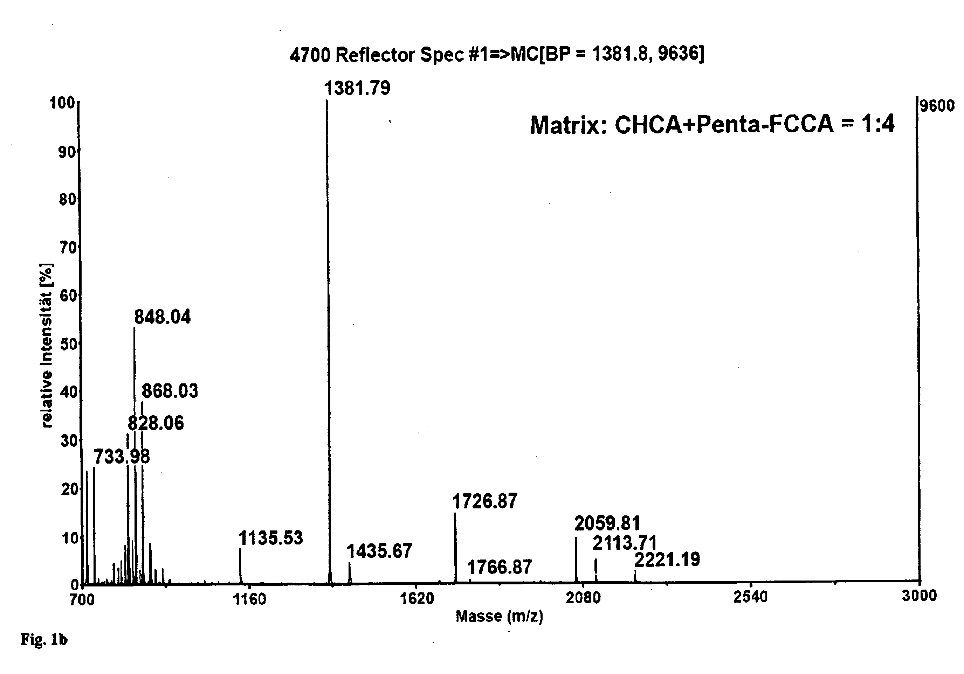

Weitere Merkmale und Vorteile der Erfindung ergeben sich aus der folgenden detaillierten Beschreibung bevorzugter Ausführungsformen in Verbindung mit den beigefügten Zeichnungen, in denen
Fig. 1a einen Ausschnitt (oben) eines im Negativ-Ionen-Modus aufgenommenen Massenspektrums eines tryptischen β-Casein-Verdaus aufgenommen mit CHCA sowie eine entsprechende Vergrößerung (unten) auf die Analyt-Fragmente und Fig. 1b den entsprechenden Ausschnitt eines im Negativ-Ionen-Modus aufgenommenen Massenspektrums eines tryptischen β-Casein-Verdaus aufgenommen unter Verwendung mit einer Matrixmischung CHCA + α-Cyano-2,3,4,5,6-pentafluorzimtsäure (Penta-FCCA) = 1:4 zeigt;

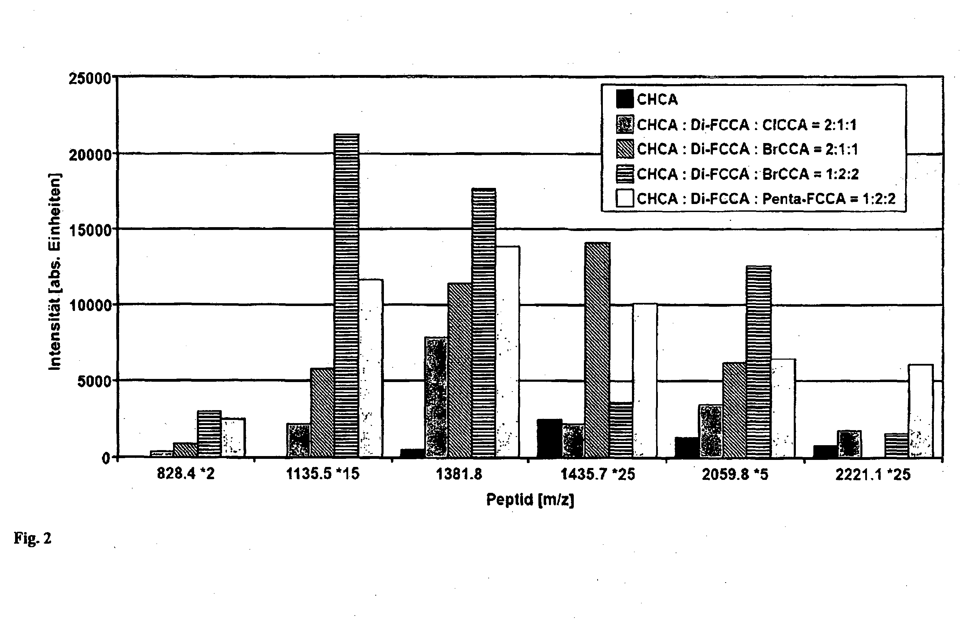

Fig. 2 ein Diagramm absoluter Signalintensitäten von sechs tryptischen β-Casein-Fragmenten, aufgenommen im Negativ-Ionen-Modus unter Verwendung von CHCA sowie verschiedener ternärer Matrix-Systeme unter Beteiligung von erfindungsgemäß verwendeter Matrizes und wiedergegeben durch ihr jeweiliges m/z-Verhältnis, zeigt;

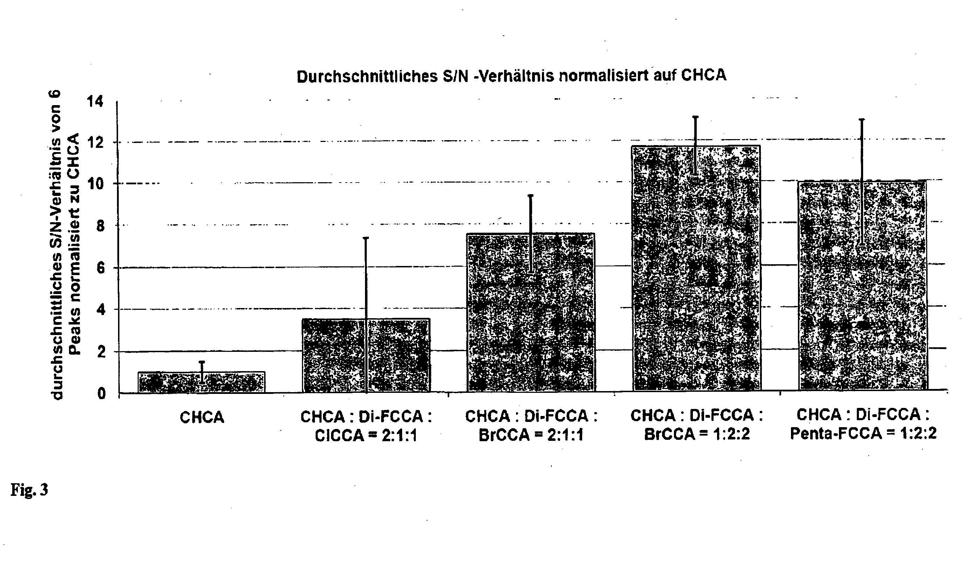

Fig. 3 die gemittelten Signal-zu-Rauschen (S/N)-Verhältnisse aller in Figur 2 analysierten Peptide in Abhängigkeit der verwendeten Matrix beziehungsweise -mischung zeigt, wobei die S/N-Verhältnisse auf CHCA normiert wurden;

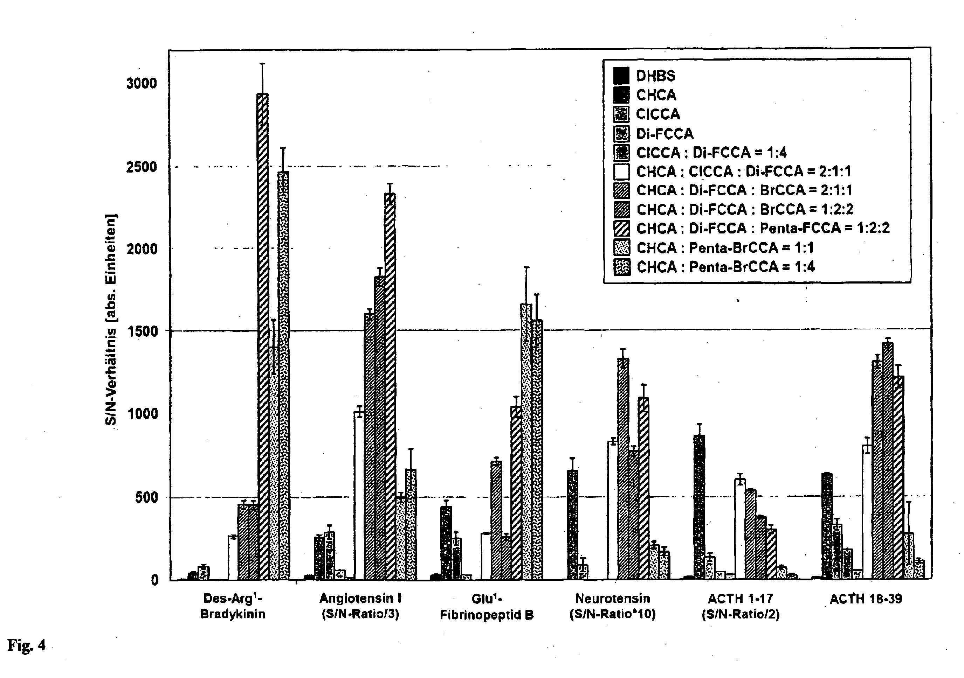

Fig. 4 ein Diagramm von im Negativ-Ionen-Modus erzielten S/N-Verhältnissen verschiedener konventioneller Matrizes und Matrixmischungen unter Verwendung erfindungsgemäß verwendeter Matrizes zeigt, wobei als Analyte Peptide einer Standard-Kalibrationsmischung verwendet wurden und die dargestellten Messwerte Mittelwerte aus 10 unabhängigen Einzelmessungen darstellen. Zur einheitlichen Darstellung innerhalb des Diagramms wurden die S/N-Verhältnisse der einzelnen Analyten mit einem konstanten Faktor skaliert;

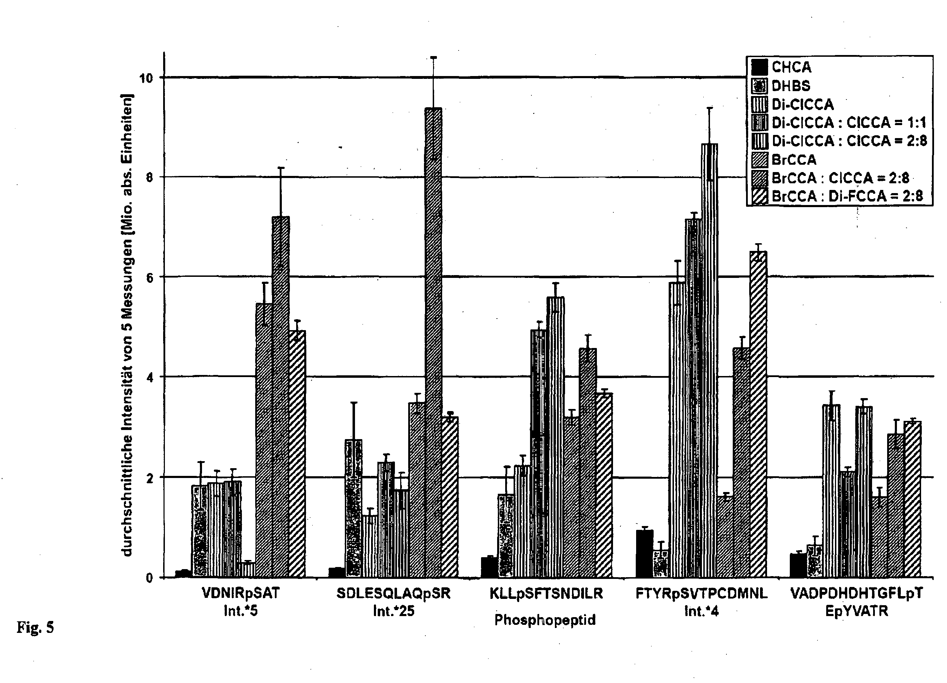

Fig. 5 ein Diagramm von im Negativ-Ionen-Modus erzielten Intensitäten unterschiedlicher Phosphopeptide gemessen mit unterschiedlichen Matrizes und -mischungen zeigt, wobei Durchschnittswerte von fünf unabhängigen Messungen aufgetragen wurden und zur besseren Übersichtlichkeit die Intensitäten einiger Phosphopeptide mit einem für alle Matrizes konstanten Faktor skaliert wurden, angegeben nach den jeweiligen Peptidsequenzen in der Abszisse;

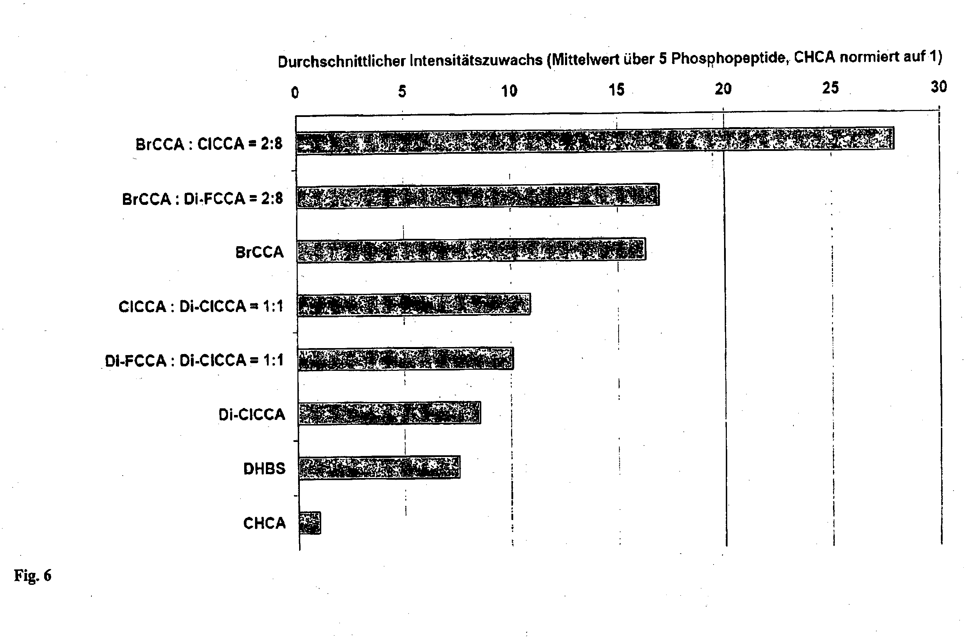

Fig. 6 ein Diagramm durchschnittlich im Negativ-Ionen-Modus erzielter Intensitäten unterschiedlicher Phosphopeptide bei Verwendung verschiedener Matrizes bzw. Matrixmischungen zeigt, wobei die mit dem Standard CHCA erzielten Signalintensitäten nach Mittelung auf 1 normiert wurden;

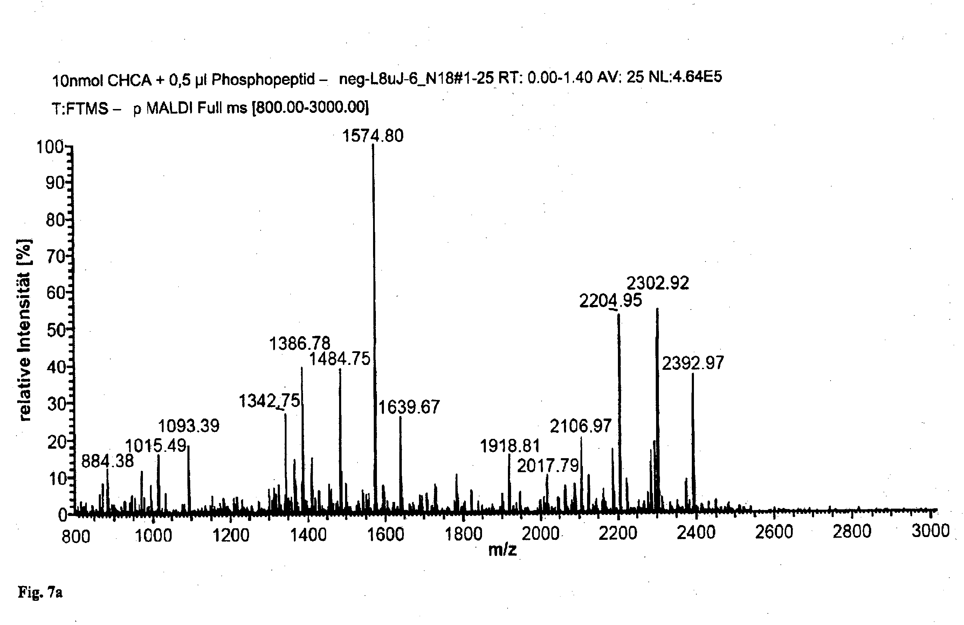

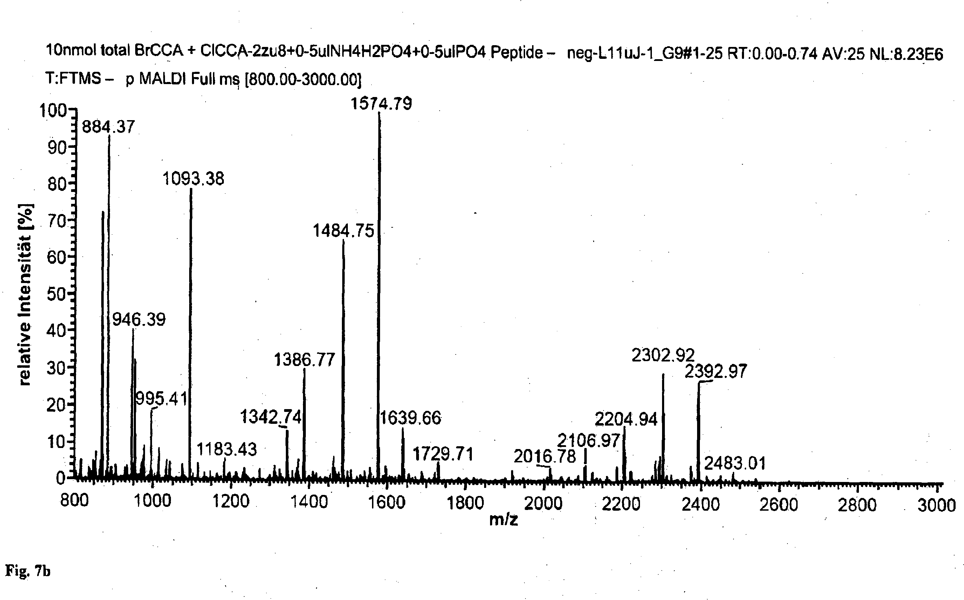

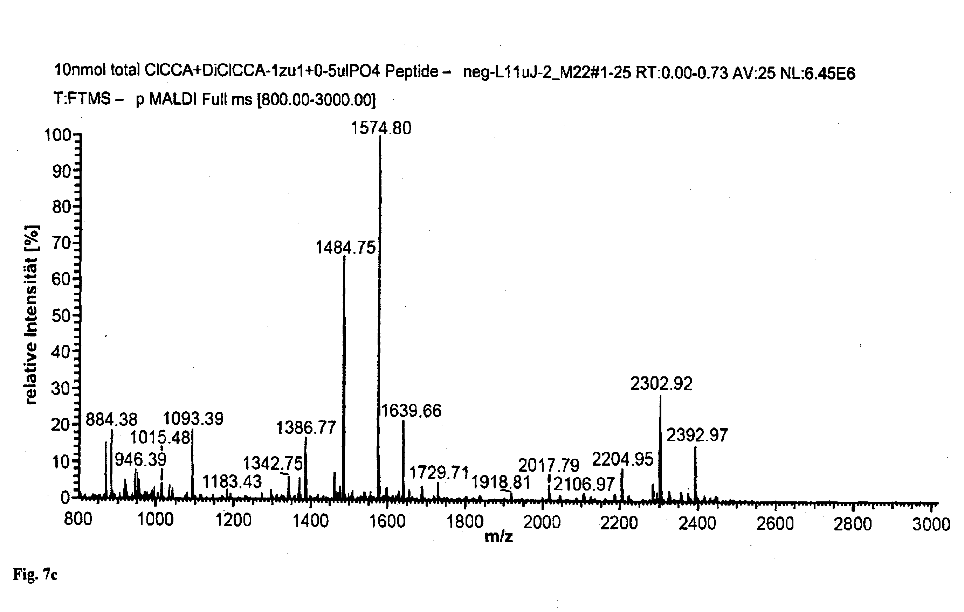

Fig. 7a-c im Negativ-Ionen-Modus aufgenommene Massenspektren zur Detektion verschiedener Phosphopeptide zeigen, mit CHCA als Matrix (Fig. 7a), mit 4-Brom-α-cyanozimtsäure (BrCCA):4-Chlor-α-cyanozimtsäure (ClCCA) = 2:8 als Matrix (Fig. 7b) und mit α-Cyano-2,4-dichlorzimtsäure (Di-ClCCA) : ClCCA = 1:1 als Matrix (Fig. 7c); und

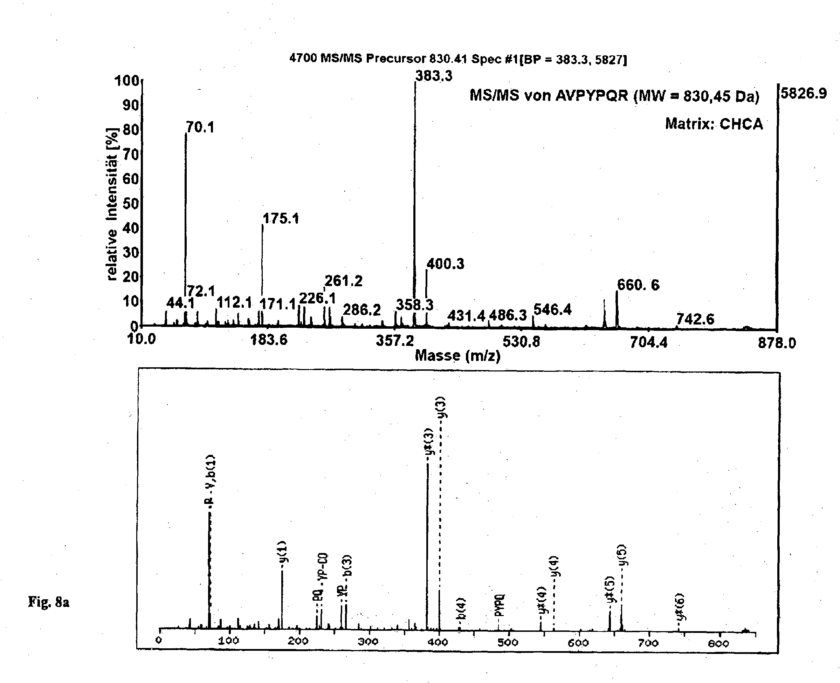

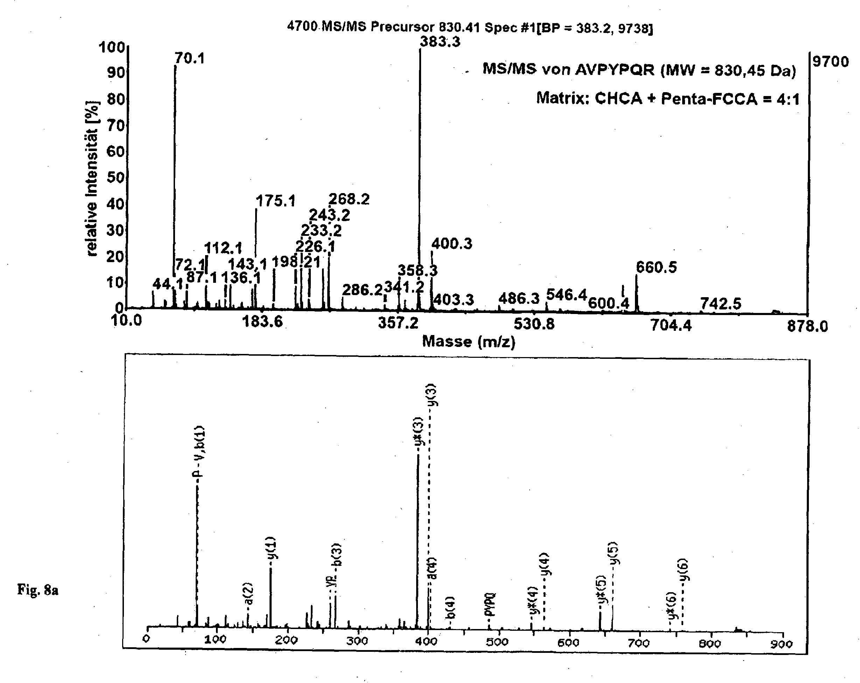

Fig. 8a-c Fragmentierungen verschiedener Peptide im Positiv-Ionen-Modus zeigen.

### Synthese der substituierten α-Cyanozimtsäurederivate

Erfindungsgemäß verwendete halogenierte Cyanozimtsäurederivate können beispielsweise durch Kondensation der substituierten Aldehyde mit Cyanoessigsäure(derivaten) gemäß einer Knoevenagel-Kondensation hergestellt werden. Die hierzu erforderlichen substituierten Benzaldehyde können im Fall von Pentahalogenderivaten aus den entsprechend halogenierten Toluolderivaten durch Oxidation mit Schwefeltrioxid dargestellt werden.

### Ausführungsbeispiel zur Darstellung von 2,3,4,5,6-Pentabrombenzaldehyd

10,1 g (0,02 mol) 2,3,4,5,6-Pentabromtoluol werden in 80 g Schwefeltrioxid gelöst und für 24 Stunden unter Wasserausschluss rückflussiert. Nach beendeter Reaktion wird überschüssiges Schwefeltrioxid unter reduziertem Druck abgetrennt. Die gebildete Dioxonium-Komponente wird durch Zugabe zu 200 ml Eis zum Aldehyden hydrolysiert. Nach kurzem Erhitzen auf 75 °C und nachfolgender Kühlung wird der Aldehyd abfiltriert, bis zur pH-Neutralität gewaschen und getrocknet. Rekristallisation aus Chlorbenzol ergibt 8,4 g (0,017 mol) 2,3,4,5,6-Pentabrombenzaldehyd als cremefarbene Nadeln. Ausbeute: 84% der Theorie.

### Ausführungsbeispiel zur Darstellung von 2,3,4,5-Tetrabrom-α-cyanozimtsäure

1,5 g 2,3,4,5-Tetrabrombenzaldehyd (MW = 421,1 g/mol; 1 Äq.; 3,56 mmol) werden mit 317,7 mg Cyanessigsäure (MW = 85,1 g/mol; 1,05 Äq.; 3,74 mmol) und 7,8 mg Piperidiniumacetat (MW = 145,2 g/mol; 0,015 Äq.; 0,05 mmol) in 30 ml trockenem Toluol für 1,5 Stunden rückflussiert. Ein Wasserabscheider dient der Abtrennung des Kondensationswassers. Nach Abkühlen auf Raumtemperatur wird das Produkt abfiltriert und mit reichlich kaltem Wasser gewaschen. Das Rohprodukt wird wiederholt aus einem Methanol/Wasser-Gemisch umkristallisiert. Nach Abfiltrieren und Trocknen im Vakuum erhält man 1,65 g α-Cyano-2,3,4,5-tetrabromzimtsäure. Ausbeute: 95% der Theorie.

### Ausführungsbeispiel zur Darstellung von α-Cyano-2,3,4,5,6-pentafluorzimtsäure

1 Äq. Cyanessigsäure (n = 12,95 mmol; m = 1,10g), 0,9 Äq. 2,3,4,5,6-Pentafluorbenzaldehyd (MW = 196,07 g/mol; n = 11,66 mmol; m = 2,287 g) und 0,012 Äq Piperidiniumacetat (n = 0,156 mmol, m = 22,55 mg) werden für 3 Stunden in ausreichender Menge Toluol rückflussiert. Nach Kühlen auf 40 °C werden die hellgelben Kristalle abfiltriert und mit kaltem Wasser gewaschen. Rekristallisation aus Methanol/Wasser gibt ein gelbes Öl, das abgetrennt und mit kaltem HPLC-Wasser gewaschen wird, woraufhin Kristallisation erfolgt. Lösen der Kristalle in Methanol und Ausfällen durch Zugabe des doppelten Volumens an kaltem Wasser lässt das Produkt direkt kristallin ausfällen. Ausbeute: 62% der Theorie.

Sollte ein bestimmtes Produkt nach beendeter Reaktion beim Abkühlen nicht kristallisieren, wird unter Vakuum bis zur beginnenden Kristallisation eingeengt und der Rückstand mit kaltem Wasser gewaschen. Um ein Ausölen bei Komponenten wie beispielsweise α-Cyano-2,3,4,5,6-pentafluorzimtsäure mit Schmelzpunkten unterhalb der Temperatur, bei der das Löslichkeitsprodukts unterschritten wird, zu verhindern, wird das Derivat in diesen Fällen durch schnelle Zugabe ausreichender Mengen an kaltem HPLC-Wasser zu der klaren methanolischen Lösung ausgefällt. Einige Derivate wie beispielsweise 2,3,6-Trichlor-α-cyanzimtsäure oder 2,3,4,5-Tetrachlor-α-cyanzimtsäure dimerisieren bei zu starker Energiezufuhr zu einem gelben in Toluol gering löslichen Öl, welches in der Kälte kristallisiert. Zur Darstellung dieser Komponenten wird der Katalysatoranteil auf 0,03 Äq. erhöht und die Reaktionszeit auf 1 Stunde begrenzt, mittels DC kann auf vollständige Umsetzung getestet werden. Nach Abkühlen auf 50 °C wird die Toluolphase vom öligen Nebenprodukt abgetrennt und das gewünschte Produkt durch weiteres Abkühlen ausgefällt. Da Rekristallisation ebenfalls mit Dimerisierung und Ausbeuteverlusten einhergeht, wird das Rohprodukt zügig in Methanol gelöst und durch Zugabe ausreichender Mengen kalten Wassers schnell ausgefällt.

Die Cyanozimtsäurederivate können durch übliche Verfahren mit den Analyten vermischt werden, um eine geeignete Probe für die MALDI-Massenspektrometrie bereitzustellen.

Ein beispielhaftes Verfahren zur Vermischung ist beispielsweise das Dried Droplet-Verfahren. Dabei werden Matrix und Analyt gelöst und zeitgleich (durch Vormischen) oder nacheinander auf einer beliebigen Oberfläche appliziert. Durch Verdunsten des Lösungsmittels erfolgt Kristallisation der Matrix unter Einschluß der Analytverbindungen.

Ferner kann das Surface Preparation-Verfahren eingesetzt werden, bei dem die Matrix bzw. das Matrixgemisch gelöst und ohne Analyt auf einer beliebigen Oberfläche appliziert wird. Durch Verdunsten des Lösungsmittels erfolgt die (Ko-)Kristallisation der Matrixverbindung(en). Der gelöste Analyt wird auf die kristalline Matrix aufgetragen, wobei durch Auflösen lediglich oberflächennaher Matrixschichten bei Rekristallisation ein Einschluß der Analytverbindung in aufkonzentrierter Form erfolgt.

Das Sublimations-Verfahren entspricht dem Verfahren der Surface Preparation mit dem Unterschied, dass die Matrix nicht aus einer Lösung auskristallisiert, sondern unter Sublimation durch Abscheidung aus der Gasphase auf eine Oberfläche aufgetragen wird.

Schließlich ist ein sogenanntes Airbrush-Verfahren möglich, bei dem Matrix und Analyt in einem gemeinsamen Lösungsmittel(gemisch) gelöst und durch eine Sprühvorrichtung dispers verteilt werden (Aerosolbildung). Durch die große Oberfläche erfolgt eine rasche Verdunstung des Lösungsmittels unter Ausbildung kleiner Matrix-Analyt-Kristalle. Alternativ kann die Matrix auch ohne Analyt gelöst werden und auf zu untersuchende Oberflächen (zum Beispiel Gewebeabschnitte) gesprüht oder anderweitig appliziert werden.

Eine neuartige Anwendungsmöglichkeit stellt die Präparation der Matrix als ionische Flüssigkeit dar: Zu diesem Zweck wird die gelöste Matrix mit äquimolarer Menge an Base, wie Pyridin oder Diethylamin, versetzt und einrotiert, wodurch sich ein flüssiger ionischer Matrixfilm bildet, der mit der Analytlösung auf beliebigen Oberflächen appliziert werden kann.

Im Folgenden wird unter einem "Verdau" ein an bestimmten Aminosäurepositionen enzymatisch geschnittenes Protein verstanden werden, wodurch viele kleinere Peptide entstehen.

Für die durchgeführten Untersuchungen wurden ein ABI 4800 MALDI TOF/TOF™ Analyzer im MS- und MS/MS-Modus bei 355 nm sowie eine MALDI LTQ Orbitrap XL im Fourier-Transform-Massenspektrometrie-Modus und einer Laserwellenlänge von 337 nm verwendet.

Das Massenspektrometer trennt unterschiedliche Ionenarten (Analytionen, Matrixionen) nach ihrem Masse/Ladungsverhältnis auf. Normalerweise tragen die Ionen in MALDI nur eine (positive oder negative) Ladung, so dass bei Ladung = 1 das Masse/Ladungsverhältnis gleich der Masse der Ionen ist. Die Abszisse der Spektren gibt das Massen/Ladungsverhältnis (und somit in annähernd allen Fällen die Masse) der Ionen wieder, wobei die Einheit Dalton bzw. g/mol ist.

Stärkere Signale - die durch senkrechte Striche in den Spektren wiedergegeben sind, wobei die Höhe der Striche (Signale) mit der dieses Signal verursachenden Ionenspeziesmenge korreliert - werden einzeln mit ihrer Masse bzw. Masse/Ladungsverhältnis gekennzeichnet.

Die Ordinatenskala richtet sich nach dem stärksten Signal innerhalb des jeweiligen Spektrums und gibt den geräteabhängigen Absolutwert des stärksten Signals an. Alle anderen in dem Spektrum dargestellten Signale werden relativ zu dem stärksten Signal dargestellt (linke Ordinatenachse). Der rechte Ordinatenwert hat nur eine Aussagekraft bei Vergleichen mit weiteren Signalen innerhalb eines Spektrums oder anderen Spektren desselben Massenspektrometers.

### Beispiel 1

### Steigerung der Signalintensitäten

Durch Verwendung sensitiverer halogenierter Matrizes können die Analyt-Signalintensitäten deutlich gesteigert werden. Dies wird in Fig. 1 für einen enzymatischen Verdau des Proteins β-Casein mit der Protease Trypsin veranschaulicht: Die stärksten detektierbaren Signale bei Verwendung der Standardmatrix α-Cyano-4-hydroxyzimtsäure (CHCA) sind auf analytunabhängige Matrixeigensignale zurückzuführen (Fig. 1a, oben). Erst in der Vergrößerung werden die Peptidsignale des Proteinverdaus deutlich sichtbar (Fig. 1a, unten). Die absolute Signalintensität für das stärkste Fragment beträgt lediglich 439 Einheiten. Im Vergleich dazu ermöglicht die Zugabe der hochreaktiven Matrix α-Cyano-2,3,4,5,6-pentafluorzimtsäure (Penta-FCCA) deutlich sensitivere Messungen, vgl. Fig. 1b: Die absolute Signalintensität des stärksten Analytsignals beträgt in diesem Fall bei gleicher Analytmenge 9600 Einheiten, weiterhin sind die Analytsignale deutlich stärker detektierbar als die Matrixeigensignale im niederen Massenbereich.

### Parameter:

Massenspektrometer, ABI 4800 MALDI TOF/TOF™ Analyzer; Modus, MS; Laserwellenlänge, 355nm; Analyt, 1 pmol tryptischer β-Casein Verdau gelöst in 30% Acetonitril/0,01% Trifluoressigsäure; Polarität, Negativ; Matrix, 10 nmol der entsprechenden Matrix bzw. - mischung gelöst in 70% Acetonitril zu c = 20 mM, V = 0,5 µl.

### Beispiel 2

### Steigerung der Signalintensitäten am Beispiel ternärer Matrixmischungen

Eine Mischung aus einem in den Ansprüchen definierten Derivat und zwei weiteren Komponenten als ternäres Matrixsystem kann ebenfalls zur Sensitivitätssteigerung erfindungsgemäß verwendet werden. Dies wird in Fig. 2 anhand der absoluten Signalintensitäten von sechs tryptischen β-Casein Peptid-Fragmenten verdeutlicht. Die hierfür verwendete Protease Trypsin wies eine zusätzliche chymotryptische Aktivität auf, was durch weitere Schnittmöglichkeiten nach z.B. Phenylalanin die Generierung des Peptids *m*/*z* = 1381,79 Da (deprotoniert) mit der Sequenz (F)LLYQEPVLGPVR(G) oder dem oxidierten Peptid (F)LQPEVMGVSKVKEAMAPKHK(E) mit *m*/*z* = 2221,19 Da (deprotoniert) ermöglicht. Aufgetragen sind die abs. Intensitäten der jeweiligen Peptide, wiedergegeben anhand ihrer *m*/*z*-Verhältnisse und in Abhängigkeit der verwendeten Matrix bzw. -mischung. Zur Abbildung innerhalb eines Diagramms wurden die Signalintensitäten der jeweiligen Peptide mit einem konstanten Faktor multipliziert, der dem *m*/*z*-Verhältnis nachfolgend in der Abszisse vermerkt ist. Die verwendeten Matrixverhältnisse beziehen sich auf die entsprechenden Stoffmengenverhältnisse.

Es ist klar ersichtlich, dass bei Verwendung der Referenzmatrix CHCA nur sehr niedrige Signalintensitäten erzielbar sind, s. Fig. 2. Die ternäre Matrixmischung CHCA : α-Cyano-2,4-difluorzimtsäure (Di-FCCA) : 4-Chlor-α-cyanozimtsäure (ClCCA) = 2:1:1 ermöglicht in den meisten Fällen stärkere Signalintensitäten als die reine CHCA Matrix. Verwendung von 4-Brom-α-cyanozimtsäure (BrCCA) oder Penta-FCCA zur Ausbildung ternärer Mischungen ermöglicht jedoch eine noch deutlich sensitivere Detektion der Analyte.

Die Zunahme der Analytsensitivität durch Verwendung hochhalogenierter α-Cyanozimtsäuren bzw. von BrCCA wird weiterhin durch Fig. 3 klar ersichtlich, in der die Signal-zu-Rauschen (S/N) -Verhältnisse der in Fig. 2 analysierten Peptide bestimmt und für einen besseren Vergleich zu einem Durchschnittswert gemittelt und auf CHCA normiert wurden. Das S/N-Verhältnis gibt an, wie viel mal stärker ein Signal als das Rauschen des Spektrums in der jeweiligen Signaluingebung ist und ist daher ein Maß für die Qualität eines Signals, da an der Signalintensität selbst nicht die durch z.B. elektronisches Rauschen des Detektors verursachte Rauschstärke abgelesen werden kann. Wie aus Fig. 3 hervorgeht, sind erfindungsgemäße ternäre Mischungen als Matrizes deutlich sensitiver als bisherige Matrizes bzw. -mischungen.

### Parameter:

Massenspektrometer, ABI 4800 MALDI TOF/TOF™ Analyzer; Modus, MS; Laserwellenlänge, 355nm; Analyt, 1 pmol tryptischer β-casein Verdau gelöst in 30% Acetonitril/0,01% Trifluoressigsäure; Polarität, Negativ; Matrix, 10 nmol der entsprechenden Matrix bzw.-mischung gelöst in 70% Acetonitril zu c = 20 mM, V = 0,5 µl.

### Beispiel 3

Ein weiteres Beispiel für die Erhöhung der Analytsensitivität unter Verwendung einer Standardpeptid-Kalibrationsmischung ist in Fig. 4 wiedergegeben. Gemessen wurde erneut im Negativ-Ionen Modus mit verschiedenen Matrizes und -mischungen, analysiert wurde das S/N-Verhältnis der in der Kalibrationsmischung enthaltenen Peptide. Als Analyte wurden Peptide eines Standard-Kalibrationsmix verwendet. Als Referenzen dienten eine 9:1 (n/n) Mischung aus 2,5-Dihydroxybenzoesäure und 2-Hydroxy-5-methoxybenzoesäure, weiterhin CHCA, ClCCA, Di-FCCA sowie eine Mischung aus ClCCA und Di-FCCA mit und ohne Zugabe von CHCA. Als beispielhafte Vertreter der erfindungsgemäß verwendeten Zimtsäurederivate wurden BrCCA, Penta-FCCA und α-Cyano-2,3,4,5,6-pentabromzimtsäure (PentaBrCCA) ausgewählt. Wie aus Fig. 4 ersichtlich ist, wird bei erfindungsgemäßer Verwendung von Matrixmischungen ein deutlicher Zugewinn an Sensitivität erzielt.

### Parameter:

Massenspektrometer, ABI 4800 MALDI TOF/TOF™ Analyzer; Modus, MS; Laserwellenlänge, 355nm; Analyt, 0,5 µl Sequazyme Mass Standard Kit (Mix 1+2) gelöst in 30% Acetonitril/0,01% Trifluoressigsäure; Polarität, Negativ; Matrix, 10 nmol der entsprechenden Matrix bzw. -mischung gelöst in 70% Acetonitril zu c = 20 mM, V = 0,5 µl, für DHBS c = 20 mg/ml in Wasser, V = 1 µl.

### Beispiel 4

### Steigerung der Detektionssensitivität phosphorylierter Peptide

Phosphorylierte Peptide weisen aufgrund ihrer leicht deprotonierbaren Phosphatfunktion oft deutlich geringere Ionisierungseffizienzen im Positiv-Ionen Modus als die entsprechenden nichtphosphorylierten Analyte auf (Janek et al. "Phosphopeptide analysis by positive and negative ion matrix-assisted laser desorption/ionization mass spectrometry", Rapid Communication in Mass Spectrometry (2001), Vol. 15, Seiten 1593-1599). Es bietet sich daher an, die Analyse von Phosphopeptiden im Negativ-Ionen Modus durchzuführen, sofern eine ausreichende Sensitivität zur Detektion des/r Analyten gegeben ist. Fig. 5 beinhaltet einen Vergleich der Verwendung verschiedener konventioneller sowie erfindungsgemäßer Matrizes in Bezug auf die Detektionsempfindlichkeit beispielhafter Phosphopeptide. Die Phosphorylierungsstelle ist in den in der Abszisse von Fig. 5 aufgeführten Sequenzen durch ein der entsprechenden Aminosäure vorangestelltes "p" gekennzeichnet. Die standardmäßig verwendeten Matrices CHCA und DHBS sind den Derivaten α-Cyano-2,4-dichlorzimtsäure (Di-ClCCA) und BrCCA gegenübergestellt, die ohne weitere Zusätze oder in Form von binären Matrixsystemen präpariert wurden. Das reine oder in Kombination mit weiteren Matrizes präparierte BrCCA-Derivat erlaubt für alle Analyte eine deutlich sensitivere Detektion. Das Di-CICCA-Derivat weist ohne oder mit Zusatz weiterer Matrizes besonders hohe Empfindlichkeiten für höhermolekulare phosphorylierte Analyte auf.

### Parameter:

Massenspektrometer, MALDI LTQ Orbitrap XL; Modus, Fourier-Transform-Massenspektrometrie; Laserwellenlänge, 337nm; Analyt, 1 µl synthetischer Phosphopeptid-Mix gelöst in 30% Acetonitril/0,01% Trifluoressigsäure; Polarität, Negativ; Matrix, 10 nmol der entsprechenden Matrix bzw. -mischung gelöst in 70% Acetonitril zu c = 20 mM, V = 0,5 µl, für DHBS c = 20 mg/ml in Wasser, V = 1 µl.

Eine matrixspezifische Mittelung der erzielten Durchschnittsintensitäten aus Fig. 5 über alle Phosphopeptide mit nachfolgender Normierung auf den Standard CHCA erlaubt einen schnellen Überblick über die Leistungsfähigkeit bzw. Sensitivität der unterschiedlichen Derivate und ihrer Mischungen und verdeutlicht den starken Zugewinn an Analyt-Signalstärke für die neu entwickelten Derivate, s. Fig. 6.

Beispielhafte Spektren der für Fig. 5 und 6 zugrundeliegenden Daten mit den entsprechenden Steigerungen der Absolutintensitäten sind für CHCA (abs. Intensität 464.000 Einheiten), BrCCA : ClCCA = 2:8 (abs. Intensität 8.230.000 Einheiten) und Di-ClCCA : ClCCA = 1:1 (abs. Intensität 6.450.000 Einheiten) in den Spektren der Fig. 7a-c wiedergegeben.

### Beispiel 5

### Stärkere Fragmentierung bei MS/MS-Analysen

MS/MS-Analysen dienen der Strukturverifizierung von Analyten. Zu diesem Zweck wird der zu fragmentierende Analyt über einen Precursorfilter isoliert und nachfolgend über z.B. Stoßgas fragmentiert. Die gebildeten Fragmente erlauben Aussagen über die Analytstruktur, z.B. die Aminosäureabfolge bei Peptiden sowie mögliche posttranslationale Modifikationen. Aufgrund einer Vielzahl von möglichen Bindungsbrüchen entsteht üblicherweise eine große Anzahl von Fragmenten, wodurch die ursprüngliche Gesamtintensität eines Signals auf sehr viele Fragmentsignale aufgeteilt wird, was mit deutlichen Intensitätsverlusten einhergeht. MS/MS-Spektren weisen daher oft nur schwache Intensitäten auf, weshalb viele Fragmente nicht detektierbar sind oder die entsprechenden MS/MS-Spektren sind bei schwachen Precursorn gänzlich unaussagekräftig. Es ist daher äußerst hilfreich, wenn durch sensitivere Matrizes höhere Precursor-Intensitäten erzielt und damit nachfolgend aussagekräftigere Fragmentspektren generiert werden können.

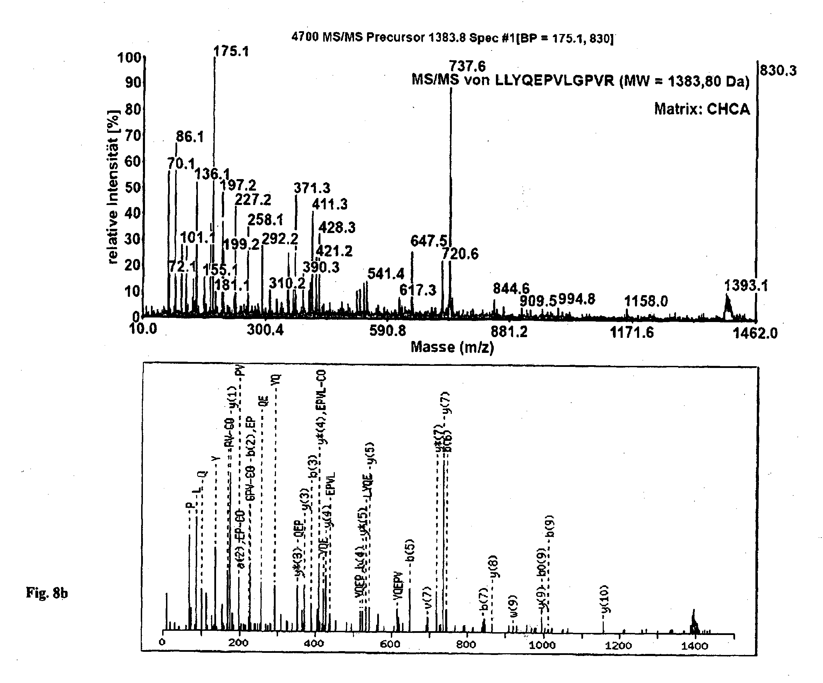

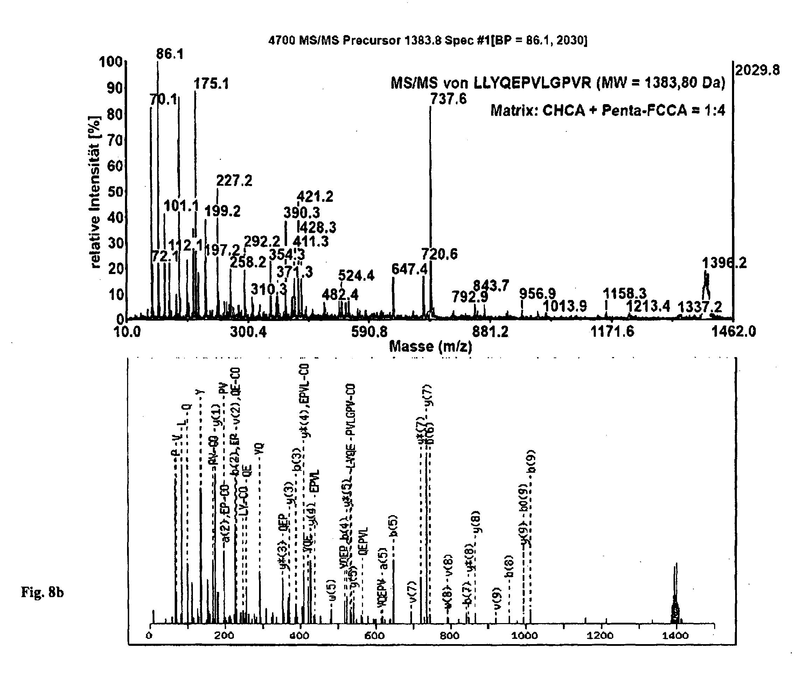

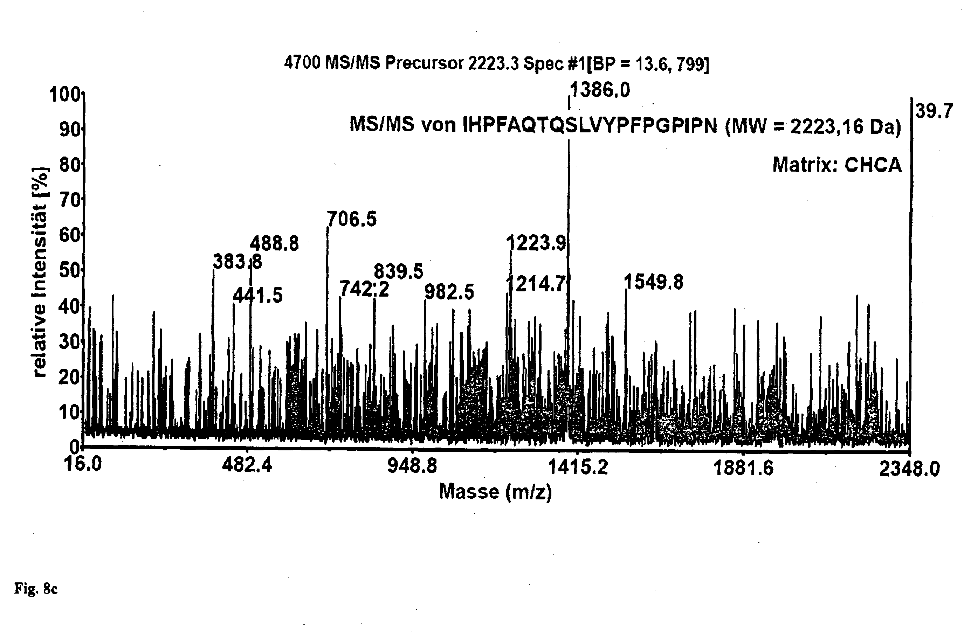

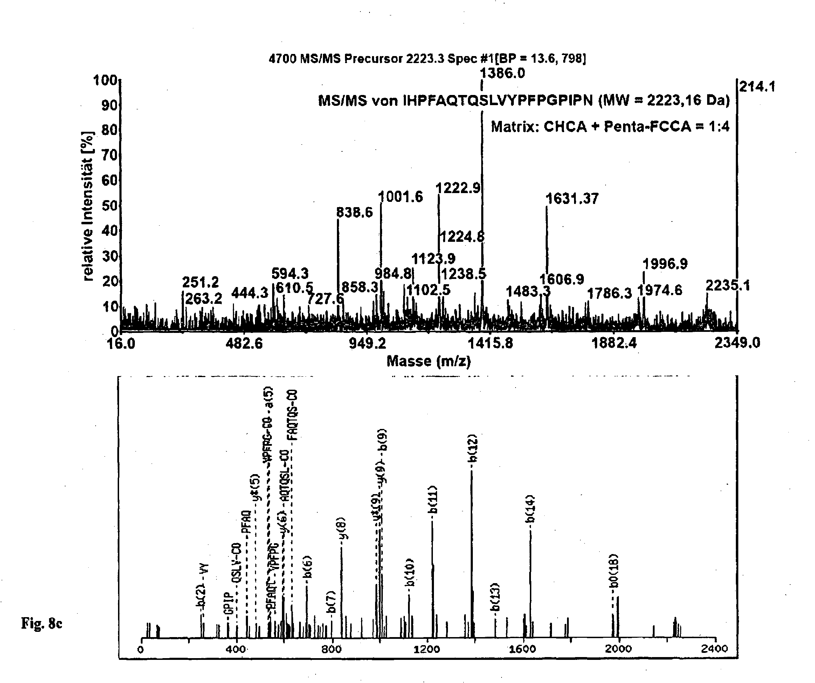

Die verstärkte Fragmentierung mit intensiveren und zahlreicheren Fragmenten durch Verwendung von Penta-FCCA wird aus Fig. 8a-c ersichtlich. Die aufgenommenen Originalspektren enthalten die gemessenen Signal-m/z-Verhältnisse, wiedergegeben durch waagerechte Zahlenwerte, die Resultate der automatischen Spektrenanalysen durch die Online-Suchmaschine Mascot (www.matrixscience.com) sind unter den Originalspektren abgebildet und enthalten die Annotationen der erkannten Fragmente anhand der von Johnson, Martin und Biemann vorgeschlagene Nomenklatur (Johnson et al., "Collision-induced fragmentation of (M+H)+ ions of peptides. Side chains specific sequence ions", International Journal of Mass Spectrometry and Ion Processes (1988), Band 86, Seiten 173-174): Bereits bei 20% Stoffmengenzugabe von Penta-FCCA sind starke Zunahmen der Fragmentsignalintensitäten im Vergleich zur Referenz CHCA erzielbar, vergleiche die abs. Intensitäten von Fig. 8 (8a: CHCA, 5827; CHCA+Penta-FCCA, 9700; 8b, CHCA, 830; CHCA+Penta-FCCA, 2030; 8c, CHCA, 40; Penta- FCCA, 214). Dies ermöglicht sowohl Detektion einer höheren Anzahl von Fragmenten (vgl. Fig. 8c) als auch von intensiveren Fragmenten (vgl. Fig. 8a, b), wodurch zum einen die Wahrscheinlichkeit einer erfolgreichen Strukturaufklärung ansteigt und zum anderen höhere Intensitäten bei nachfolgenden MS³-Experimenten, d.h. weitergehenden Zerfallsanalysen der gebildeten Fragmente, erzielt werden können.

In Fig. 8c konnte für das mit der Referenzmatrix CHCA-aufgenommene Spektrum (ganz oben) aufgrund der geringen Intensität der erzielten Signale keine automatische Fragmentanalyse und -zuordnung durchgeführt werden. Im Gegensatz dazu weist das untere Spektrum in Fig. 8c, aufgenommen mit einer erfindungsgemäßen Matrix, stärkere Signale auf, so dass eine automatische Zuordnung vorgenommen werden konnte.

### Parameter:

Massenspektrometer, ABI 4800 MALDI TOF/TOF™ Analyzer; Modus, MS/MS; Laserwellenlänge, 355nm; Analyt, 1 pmol tryptischer β-casein Verdau; Polarität, Positiv

## Patentansprüche

1. Verwendung eines halogenierten Cyanozimtsäurederivats der allgemeinen Formel: 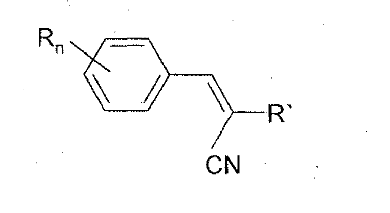 wobei R unabhängig ausgewählt ist aus F, Cl und Br, n = 3, 4 oder 5 ist und R' ausgewählt ist aus COOH, CONH₂, SO₃H und COOR" mit R" - C₁-C₅-Alkyl in einer Matrix für eine MALDI-Massenspektrometrie eines Analyten.

2. Verwendung nach Anspruch 1, **dadurch gekennzeichnet, dass** die Matrix zur MALDI-Massenspektrometrie negativer Ionen verwendet wird.

3. Verwendung nach Anspruch 1 oder 2, **dadurch gekennzeichnet, dass** n = 5 ist.

4. Verwendung nach Anspruch 1, **dadurch gekennzeichnet, dass** die Matrix zur MALDI-Massenspektrometrie α-Cyano-2,3,4,5,6-pentafluorzimtsäure ist.

5. Verwendung nach Anspruch 1, **dadurch gekennzeichnet, dass** die Matrix zur MALDI-Massenspektrometrie zusätzlich 4-Brom-α-cyanozimtsäure und/oder α-Cyano-2,4-dichlorzimtsäure enthält.

6. Verwendung nach einem der vorangehenden Ansprüche, **dadurch gekennzeichnet, dass** der Analyt ausgewählt ist aus Protein, Peptid, Polynukleinsäure, Lipid, phosphorylierter Verbindung, Saccharid, Arzneistoff, Metabolit, synthetischem und natürlichem (Ko)-Polymer und anorganischer Verbindung.

7. Verwendung nach einem der vorangehenden Ansprüche, **dadurch gekennzeichnet, dass** die Matrix mit dem Analyten vermischt wird.

8. Verwendung nach Anspruch 7, **dadurch gekennzeichnet, dass** das molare Mischungsverhältnis von Analyt zu Matrix 1:100 bis 1:1000000000, bevorzugt 1:10000, beträgt.

9. Verwendung nach einem der vorangehenden Ansprüche, **dadurch gekennzeichnet, dass** eine Mischung zumindest eines in Anspruch 3 genannten halogenierten Cyanozimtsäurederivats mit zumindest einem weiteren Matrixmaterial eingesetzt wird.

10. Verwendung nach Anspruch 9, **dadurch gekennzeichnet, dass** das weitere Matrixmaterial ausgewählt ist aus α-Cyano-4-hydroxyzimtsäure, α-Cyano-2,4-difluorzimtsäure, 2,5-Dihydroxybenzoesäure, Sinapinsäure, Ferulasäure, 2-Aza-5-thiothymin, 3-Hydroxypicolinsäure und 4-Chlor-α-cyanozimtsäure.

11. Verwendung nach einem der vorangehenden Ansprüche, **dadurch gekennzeichnet, dass** die Matrix mit einem inerten Füllstoff vermischt ist.

12. Verwendung nach einem der vorangehenden Anspruche, **dadurch gekennzeichnet, dass** die Matrix als ionische Flüssigkeit vorliegt.

13. Matrix für eine MALDI-Massenspektrometrie eines Analyten, welche ein halogeniertes Cyanozimtsäurederivat der allgemeinen Formel: 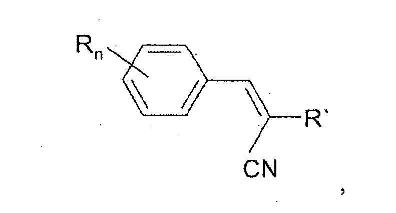 wobei R unabhängig ausgewählt ist aus F, Cl und Br, n = 3, 4 oder 5 ist und R' ausgewählt ist aus COOH, CONH₂, SO₃H und COOR" mit R" = C₁-C₅-Alkyl umfasst.

## Claims

1. Use of a halogenated cyanocinnamic acid derivative of the general formula: 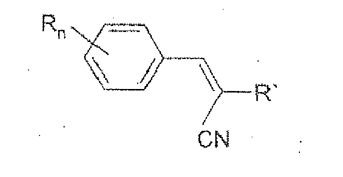 where R is independently selected from among F, Cl and Br, n = 3, 4 or 5, and R' is selected from COOH, CONH₂, SO₃H and COOR" with R" - C₁-C₅ alkyl in a matrix for MALDI mass spectrometry of an analyte.

2. Use according to claim 1, **characterized in that** negative ions are used for the matrix for the MALDI mass spectrometry.

3. Use according to claim 1 or 2, **characterized in that** n = 5.

4. Use according to claim 1, **characterized in that** the matrix for the MALDI mass spectrometry is α-cyano-2,3,4,5,6-pentafluoro cinammic acid.

5. Use according to claim 1, **characterized in that** the matrix for the MALDI mass spectrometry additionally contains 4-bromo-α-cyanocinnamic acid and/or a-cyano-2,4-dichlorocinnamic acid.

6. Use according to one of the preceding claims, **characterized in that** the analyte is selected from protein, peptide, polynucleic acid, lipid, phosphor compound, saccharide, pharmaceutical substance, metabolite, synthetic and natural (co)polymer and inorganic compounds.

7. Use according to one of the preceding claims, **characterized in that** the matrix is mixed with the analyte.

8. Use according to claim 7, **characterized in that** the molar mixing ratio of analyse to matrix is 1:100 to 1:1000000000, preferably 1:10000.

9. Use according to one of the preceding claims, **characterized in that** a mixture is used of at least one of the halogenated cyanocinnamic acid derivatives referred to in claim 1 with at least one further matrix material.

10. Use according to claim 9, **characterized in that** the further matrix material is selected from α-cyano-4-hydroxycinnamic acid, α-cyano-2,4-difluorocnnamic acid, 2,5-dihydroxybenzoic acid, sinapic acid, ferulic acid, 2-aza-5-thiothymine, 3-hydroxypicolinic acid and 4-chloro-α-cyanocinnamic acid.

11. Use according to one of the preceding claims, **characterized in that** die matrix is mixed with an inert filler substance.

12. Use according to one of the preceding claims, **characterized in that** the matrix is present as an ionic liquid.

13. Matrix for MALDI mass spectrometry of an analyte, which comprises a halogenated cyanocinnamic acid derivative of the general formula: 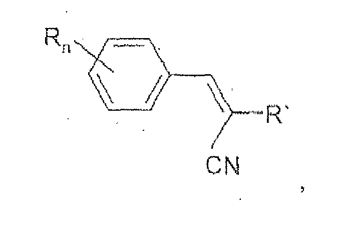 where R is independently selected from F, Cl and Br, n = 3, 4 or 5, and R' is selected from COOH, CONH₂, SO₃H and COOR" with R" = C₁-C₅-alkyl.

## Revendications

1. Utilisation d'un dérivé d'acide cyanocinnamique halogéné ayant la formule générale : 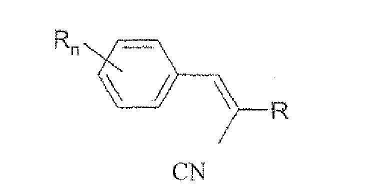 dans laquelle R est sélectionné indépendamment parmi : F, Cl et Br, n = 3, 4 ou 5 et R' est sélectionné parmi COOH, CONH₂, SO₃H et COOR" avec R"= C₁-C₅-alkyle dans une matrice pour une spectrométrie de masse MALDI (Désorption-ionisation laser assistée par matrice) d'un analyte.

2. Utilisation selon la revendication 1, **caractérisée en ce que** la matrice est utilisée pour une spectrométrie de masse MALDI d'ions négatifs.

3. Utilisation selon la revendication 1 ou 2, **caractérisée en ce que** n = 5.

4. Utilisation selon la revendication 1, **caractérisée en ce que** la matrice pour la spectrométrie de masse MALDI est de l'acide α-cyano-2, 3, 4, 5, 6-pentafluorocinnamique.

5. Utilisation selon la revendication 1, **caractérisée en ce que** la matrice pour la spectrométrie de masse MALDI contient de l'acide 4-bromo-α-cyanocinnamique et/ou de l'acide α-cyano-2,4-dichlorocinnamique.

6. Utilisation selon l'une quelconque des revendications précédentes, **caractérisée en ce que** l'analyte est sélectionné parmi : protéine, peptide, acide polynucléïque, lipide, composé phosphorylé, saccharide, substance médicinale, métabolite, (co)polymère synthétique et naturel et composé inorganique.

7. Utilisation selon l'une quelconque des revendications précédentes, **caractérisée en ce que** la matrice est mélangée avec l'analyte.

8. Utilisation selon la revendication 7, **caractérisée en ce que** le rapport molaire du mélange analyte/matrice est compris entre 1:100 et 1:1000000000, et de préférence de 1:10000.

9. Utilisation selon l'une quelconque des revendications précédentes, **caractérisée en ce qu'**elle emploie un mélange d'au moins un dérivé d'acide cyanocinnamique halogéné spécifié à la revendication 1 avec un matériau supplémentaire de matrice.

10. Utilisation selon la revendication 9, **caractérisée en ce que** le matériau supplémentaire de matrice est sélectionné parmi : acide α-cyano-4-hydroxycimnamique, acide α-cyano-2,4-difluorocinnamique, acide 2,5-dihydroxybenzoïque, acide sinapique, acide férulique, 2-aza-5-thiothymine, acide 3-hydroxypicolinique et acide 4-chloro-α-cyanocinnamique.

11. Utilisation selon l'une quelconque des revendications précédentes, **caractérisée en ce que** la matrice est mélangée avec une charge inerte.

12. Utilisation selon l'une quelconque des revendications précédentes, **caractérisée en ce que** la matrice est disponible sous la forme de liquide ionique.

13. Matrice pour une spectrométrie de masse MALDI d'un analyte, qui met en jeu un dérivé d'acide cyanocinnamique halogéné ayant la formule générale : 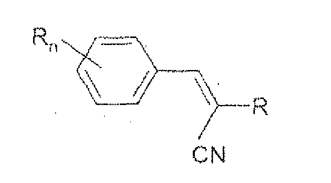 dans laquelle R est sélectionné indépendamment parmi F, Cl et Br, n = 3, 4 ou 5 et R' est sélectionné parmi : COOH, CONH₂, SO₃H et COOR" avec R" = C₁-C₅-alkyle.
